# FASCICULE DE BREVET EUROPEEN

(11) **EP 2 774 569 B1**
(45) Date de publication et mention de la délivrance du brevet: **08.07.2015**
(21) Numéro de dépôt: 14154128.4
(22) Date de dépôt: 06.02.2014
(51) Int. Cl.: A61B 19/00, F21V 21/40, H05B 33/08

(54) **Appareil d'éclairage avec une poignée de réglage de l'éclairage**
Beleuchtungsgerät mit einem Beleuchtungsreguliergriff
Lighting apparatus with a lighting-adjustment handle

(30) Priorité: 05.03.2013 FR 1351964
(43) Date de publication de la demande: 10.09.2014
(73) Titulaire: Maquet SAS, 45160 Ardon (FR)
(72) Inventeur: Comte, Lionel, 45380 La Chapelle Saint Mesmin (FR); Jousse, Robin, 45380 La Chapelle Saint Mesmin (FR); Legrand, Stéphane, 45450 Donnery (FR)
(74) Mandataire: Prugneau, Philippe

(56) Documents cités:
- EP-A1- 2 065 634
- WO-A1-2009/102192
- DE-U1- 20 316 755
- US-A1- 2006 109 650
- US-A1- 2011 095 703
- US-A1- 2012 043 915

## Description

### Domaine technique

L'invention concerne de façon générale un appareil d'éclairage et plus particulièrement un appareil d'éclairage utilisé dans un bloc opératoire pour l'éclairage d'un champ opératoire.

### Technique antérieure

De manière connue, un appareil d'éclairage comprend un module de lumière avec une ou plusieurs sources lumineuses raccordées à une alimentation électrique commandée par une unité de contrôle/commande.

Le module de lumière peut être pourvu d'une poignée pour le manipuler. Cette poignée peut en outre être montée rotative suivant son axe longitudinal pour fournir un signal de position angulaire utilisé par l'unité de contrôle/commande de l'éclairage pour régler l'éclairage, par exemple l'intensité lumineuse de l'éclairage.

La publication de brevet US 2006/0 109 650 décrit un tel appareil d'éclairage d'après le préambule de la revendication 1 dans lequel la poignée est couplée à un transducteur différentiel dont le signal électrique dépend de la position angulaire de la poignée et permet de moduler la puissance de l'éclairage en fonction de cette position. La rotation de la poignée n'est pas limitée et est dite sans fin.

De tels éclairages sont généralement portés par un bras articulé permettant de les orienter dans l'espace. Ainsi, la poignée est également utilisée par le personnel médical pour orienter l'éclairage par rapport au champ opératoire. Aussi, il est important de s'assurer que la sollicitation de la poignée pour déplacer l'éclairage ne provoque pas de manière involontaire une modulation de la puissance d'éclairage.

A cet effet, la publication de brevet EP 2 159 485 décrit un appareil d'éclairage dans lequel la rotation de la poignée provoque la variation de la dimension du champ d'éclairage, cet appareil d'éclairage comportant deux butées angulaires limitant la rotation de la poignée. Chaque butée est associée à un capteur mécanique permettant de détecter le contact entre la poignée et l'une ou l'autre des butées. La variation de la dimension du champ d'éclairage est ainsi conditionnée à cette détection de contact. De ce fait la poignée peut être utilisée pour orienter l'éclairage avec peu de risque que cela ne conduise à un changement involontaire du champ d'éclairage. Toutefois, cet agencement a des fonctionnalités qui restent limitées.

On connait aussi des documents US 2012/043915 et EP 2 065 634 des dispositifs d'éclairage médical comportant une poignée pour régler l'éclairage de façon précise. Cependant ces réglages ne dépendent pas de paramètres prédéterminés.

### Exposé de l'invention

Le but de l'invention est de remédier à ces inconvénients en proposant un appareil d'éclairage comportant une poignée dont la rotation permet de régler l'éclairage de façon précise, sans butée physique, tout en autorisant un déplacement de l'éclairage par traction sur la poignée sans risque de réglage intempestif de l'éclairage.

A cet effet, l'invention a pour objet un appareil d'éclairage, notamment pour champ opératoire, comprenant un module de lumière avec une poignée mobile en rotation et une unité de contrôle/commande qui, en réponse à une rotation de la poignée agit sur un réglage de l'appareil d'éclairage, caractérisé en ce que l'unité de contrôle/commande est agencée pour relever, à intervalles de temps réguliers successifs, une indication de position angulaire relative de la poignée et agir sur le réglage à partir des indications de position angulaire relative courante et précédente relevées respectivement pour un intervalle de temps courant et pour un intervalle de temps précédant immédiatement l'intervalle de temps courant, et en ce que l'unité de contrôle/commande est agencée pour calculer un écart angulaire courant à partir desdites indications de position angulaire courante et précédente et comparer ledit écart angulaire courant à un seuil prédéterminé.

L'idée générale à la base de l'invention est donc de régler l'éclairage à partir de la détection des positions angulaires relatives de la poignée respectivement sur deux intervalles de temps consécutifs.

L'appareil d'éclairage selon l'invention peut présenter notamment les particularités suivantes :
- l'unité de contrôle/commande est agencée pour modifier la valeur d'un paramètre de réglage de l'appareil d'éclairage quand l'écart angulaire courant est supérieur au seuil;
- l'unité de contrôle/commande est agencée pour déterminer, à chaque intervalle de temps courant, un sens pour l'écart angulaire courant et augmenter ou diminuer la valeur du paramètre de réglage en fonction du sens de l'écart angulaire courant ;
- l'unité de contrôle/commande est agencée pour comparer l'écart angulaire courant à un premier seuil prédéterminé pour un premier sens de rotation de la poignée et pour comparer l'écart angulaire courant à un second seuil prédéterminé pour un second sens de rotation de la poignée, opposé au premier sens de rotation, le premier seuil étant différent du second seuil ;
- l'unité de contrôle/commande est agencée pour détecter un changement du sens de rotation de la poignée pendant l'intervalle de temps courant et l'intervalle de tem précédent l'intervalle de temps courant et, en réponse à la détection, basculer le réglage de l'appareil d'éclairage sur d'un paramètre de réglage courant sur un paramètre de réglage prédéterminé ;
- la valeur du paramètre de réglage est une puissance d'éclairage, une température de couleur ou une dimension de tâche lumineuse ;
- l'unité de contrôle/commande bascule d'un paramètre de réglage courant à un autre paramètre de réglage suivant une liste circulaire prédéterminée de paramètres de réglage ;
- l'appareil d'éclairage comporte une roue dentée coaxiale à la poignée et liée en rotation à la poignée, un pignon agencé pour engrener avec la roue dentée, et un capteur de position angulaire relatif apte à détecter la position angulaire relative de la poignée par l'intermédiaire de la position angulaire relative du pignon ;
- le capteur de position angulaire est de type potentiomètre ;
- la poignée est creuse et apte à loger un support connecteur vidéo.

Cet agencement de l'unité de contrôle/commande permet d'éviter des modulations intempestives du ou des paramètres de réglage qui peuvent être déclenchées par une faible rotation de la poignée lorsque celle-ci est utilisée pour déplacer l'éclairage.

De plus avec cet agencement, on s'affranchit de la connaissance précise de la position angulaire absolue de la poignée en utilisant un angle de rotation relatif. Le dispositif d'éclairage selon l'invention fonctionne ainsi de manière relative et non absolue.

De plus, en réglant la valeur du ou des seuils, on peut jouer sur la sensibilité de la commande de réglage pour par exemple l'adapter à chaque utilisateur.

### Présentation sommaire des dessins

La présente invention sera mieux comprise et d'autres avantages apparaîtront à la lecture de la description détaillée d'un mode de réalisation pris à titre d'exemple nullement limitatif et illustré par les dessins annexés, dans lesquels :
- la figure 1 est une vue en perspective schématique d'un appareil d'éclairage selon l'invention utilisé dans un bloc opératoire ;
- la figure 2 est une vue en perspective d'une partie de la poignée de l'appareil d'éclairage selon l'invention,
- la figure 3 est une vue en perspective éclatée de la poignée de l'appareil d'éclairage selon l'invention illustrée dans une première configuration d'utilisation ;
- la figure 4 est une vue en perspective d'une partie de la poignée illustrée dans une seconde configuration d'utilisation ;
- les figures 5 et 6 sont des vues de dessus de la poignée de la figure 3 illustrée dans deux positions angulaires distinctes ;
- la figure 7 est une vue similaire aux figures 5 et 6 de la poignée de la figure 4 ;
- la figure 8 est une vue schématique des étapes de la phase de démarrage de l'appareil d'éclairage selon l'invention ;
- la figure 9 est une vue schématique des étapes de la nième boucle de modulation de l'éclairage au moyen de l'appareil d'éclairage selon l'invention.

### Description des modes de réalisation

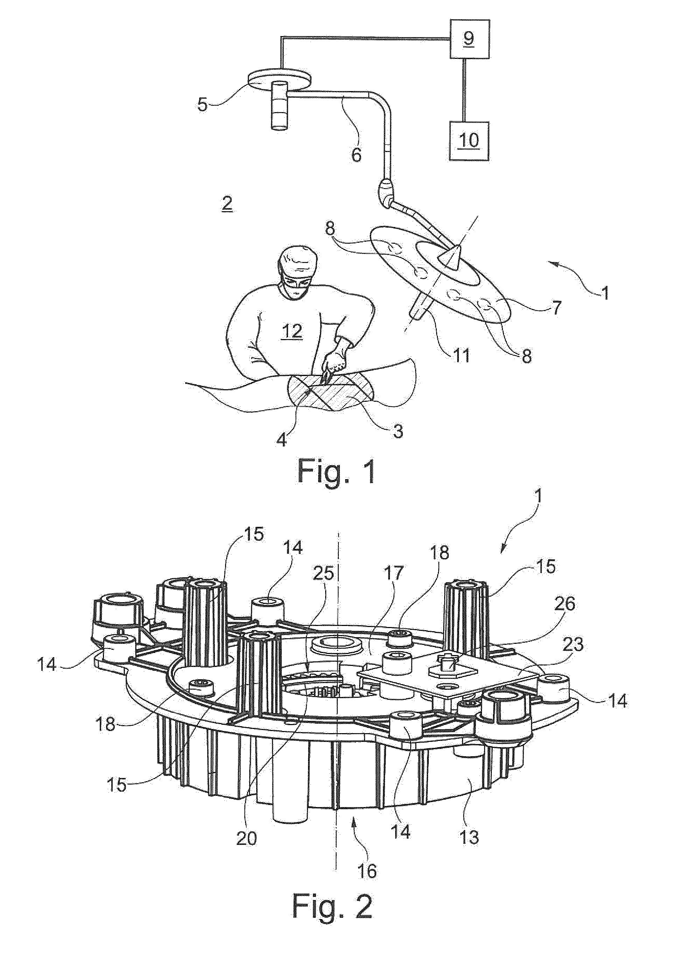

En référence à la figure 1, l'appareil d'éclairage 1 selon l'invention est notamment destiné à être utilisé dans le domaine médical, par exemple dans un bloc opératoire 2 pour former une tâche d'éclairement 3 (schématisée par des hachures) sur un champ opératoire 4.

De manière connue, l'appareil d'éclairage 1 comporte une embase 5 fixée au plafond du bloc opératoire 2 et à partir de laquelle s'étend un bras articulé 6 portant une coupole d'éclairage 7 dans laquelle sont logées des sources lumineuses 8 (schématisées en pointillé sur la figure 1), qui font partie d'un ou de plusieurs modules de lumière, telles que des LEDs (ou diodes électroluminescentes) disposées en couronne.

Les LEDs 8 sont raccordées électriquement à une alimentation électrique 9 couplée à une unité de contrôle/commande 10 apte à piloter l'alimentation électrique 9 pour moduler un ou plusieurs paramètre de réglage, par exemple la dimension de la tâche d'éclairement 3, ou encore l'intensité de la lumière produite par les LEDs 8.

L'appareil d'éclairage 1 comporte également une poignée 11, coaxiale à la couronne de LEDS 8 et s'étendant axialement dans la coupole d'éclairage 7 de sorte à pouvoir être facilement saisie par le personnel médical 12. Cette poignée 11 est portée par la coupole d'éclairage 7 par l'intermédiaire d'un support 13, visible sur les figures 2 à 4 et comportant à cet effet des orifices 14 et des tiges de fixation 15 aptes à recevoir des vis (non représentées) ou tout autre élément de fixation adapté.

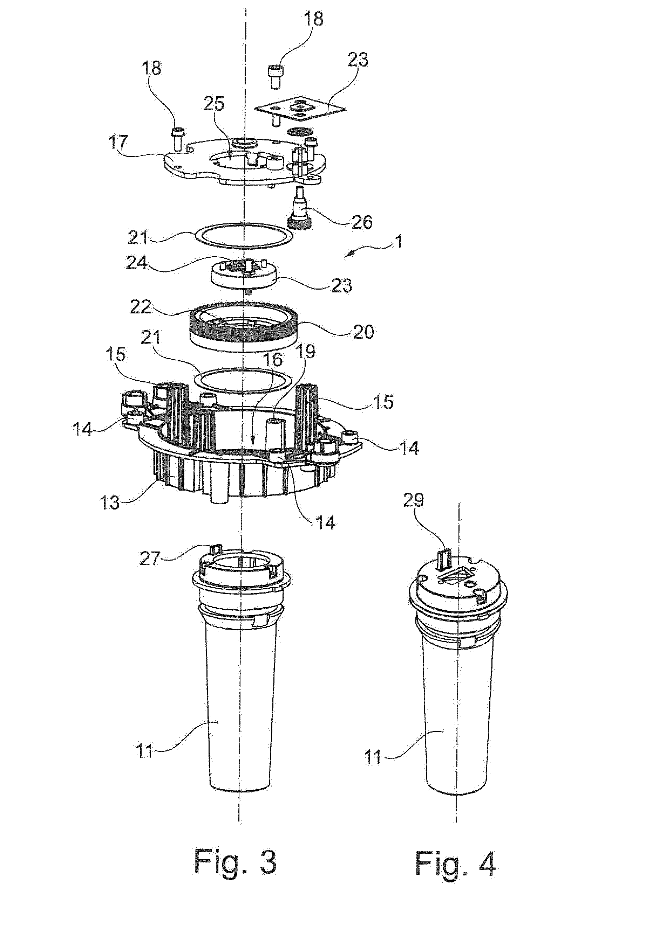

Selon l'invention et en référence aux figures 2 et 3, le support 13 présente une forme de coupelle traversée par un évidement 16 autorisant le passage de la poignée 11. La poignée 11 est montée sur le support 13 par l'intermédiaire de tout moyen adapté connu autorisant la mobilité en rotation de la poignée 11 par rapport au support 13. La coupelle est fermée par une platine 17 fixée au support 13 par exemple au moyen de vis 18 et d'orifices taraudés 19. L'appareil d'éclairage 1 selon l'invention, comporte une roue dentée 20 coaxiale à la poignée 11. Cette roue dentée 20 présente la forme d'une couronne et est logée dans la coupelle du support 13. La roue dentée 20 est séparée du support 13 et de la platine 17 par des rondelles 21 coaxiales et limitant les frottements. La roue dentée 20 présente un alésage intérieur 22 dans lequel peut être logé un support connecteur vidéo 23 apte à porter une caméra (non représentée) et pourvu de connecteurs électriques 24 pour raccorder la caméra à l'alimentation électrique 9.

Selon l'invention, la poignée 11 est creuse axialement ce qui autorise le passage de la caméra permettant de filmer le champ opératoire 4, ou de tout autre appareil électrique équivalent. La platine 17 comporte également un évidement 25 pour autoriser le passage des fils d'alimentation électrique (non représentés) de la caméra.

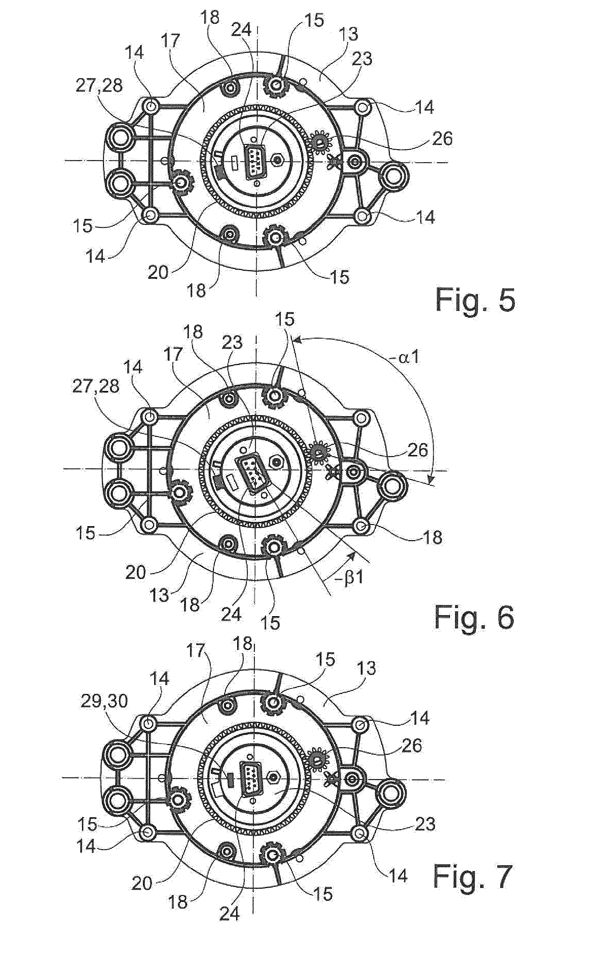

En référence aux figures 3 à 7, l'appareil d'éclairage 1 selon l'invention comporte en outre un pignon 26 logé dans la coupelle du support 13 et dont l'axe est porté par le support 13. Le pignon 26 est disposé de sorte à être dans le même plan que la roue dentée 20 avec laquelle il engrène. Le rapport entre le nombre de dents de la roue dentée 20 par rapport au nombre de dents du pignon 26 est par exemple de cinq. L'axe du pignon 26 traverse la platine 17 par un orifice prévu à cet effet. Ainsi, comme illustré par les figures 5 et 6, lorsque la poignée 11 et la roue dentée 20 tourne d'un angle-β1, le pignon 26 tourne d'un angle -α1.

En référence aux figures 3, 5 et 6, la poignée 11 est pourvue ici d'un premier ergot 27 et la roue dentée 20 est pourvue d'un premier cran 28 apte à recevoir le premier ergot 27 et à coopérer avec pour que la rotation de la poignée 11 entraine la rotation de la roue dentée 20. Cette configuration peut bien entendu être symétrique, la roue dentée comportant le premier ergot et la poignée comportant le premier cran. Cette configuration permet la modulation d'un paramètre de réglage. Dans cette configuration, le support connecteur vidéo 23 ne tourne pas avec la poignée 11. Ainsi, la poignée 11 peut être tournée sans risque d'entortillement des fils d'alimentation électrique de la caméra.

En référence aux figures 7 et 4, la poignée 11 est ici pourvue d'un second ergot 29, radialement décalé par rapport au premier ergot 27 précédent vers l'axe de la poignée 11 et le support connecteur vidéo 23 est pourvu d'un second cran 30 apte à recevoir le second ergot 29 et à coopérer avec pour que la rotation de la poignée 11 entraine la rotation du support connecteur vidéo 23 sans provoquer la rotation de la roue dentée 20. Cette configuration peut bien entendu être symétrique, le support connecteur vidéo comportant le second ergot et la poignée comportant le second cran. Cette construction permet d'utiliser un module d'éclairage selon l'invention dans une configuration selon laquelle la modulation du paramètre de réglage est obtenue par rotation de la poignée 11 et une seconde configuration selon laquelle la rotation de la poignée 11 est sans effet sur le paramètre de réglage.

L'appareil d'éclairage 1 selon l'invention comporte également un capteur de position angulaire 31 relatif (visible sur les figures 2 et 3) se présentant sous la forme d'une carte potentiomètre traversée par l'axe du pignon 26. Le capteur de position angulaire 31 est apte à émettre un signal indicatif de la position angulaire relative du pignon 26 et donc de la poignée 11. Le capteur de position angulaire 31 est traversé par un courant dont la tension varie en fonction de la position angulaire du pignon 26, par exemple de 0 à 5 volts.

Selon l'invention, l'unité de contrôle/commande 10 (ou UC) est couplée au capteur de position angulaire 31 et est programmée pour, à intervalles de temps réguliers successifs, relever des indications de position angulaire relative du pignon 26 et donc de la poignée 11, et sur la base de ces relevés successifs, moduler un paramètre de réglage particulier et/ou changer de paramètre de réglage.

Comme cela apparaitra ci-dessous, l'UC 10 répète à chaque intervalle de temps une boucle de traitement pour la modulation du paramètre de réglage courant. La durée de chaque intervalle de temps peut être par exemple de 5 ms mais peut être plus ou moins longue selon la sensibilité de réglage recherchée.

Comme indiqué plus haut, la rotation de la poignée 11 peut servir à commander un changement de commande ou de paramètre de réglage dans l'UC 10 et ceci indépendamment d'une modulation de paramètre de réglage, par exemple le passage de la commande du paramètre de réglage P1 du diamètre de la tâche d'éclairement 3 à la commande du paramètre de réglage P2 du niveau de l'éclairement visuel.

A noter, que dans l'UC 10, on peut avoir plus de deux commandes ou paramètres de réglage sélectivement actionnables, le passage d'une commande ou paramètre de réglage à une/un autre se faisant selon un ordre prédéterminé P1, P2, P3, ... Pn d'une liste circulaire prédéterminée de commandes ou de paramètres de réglage.

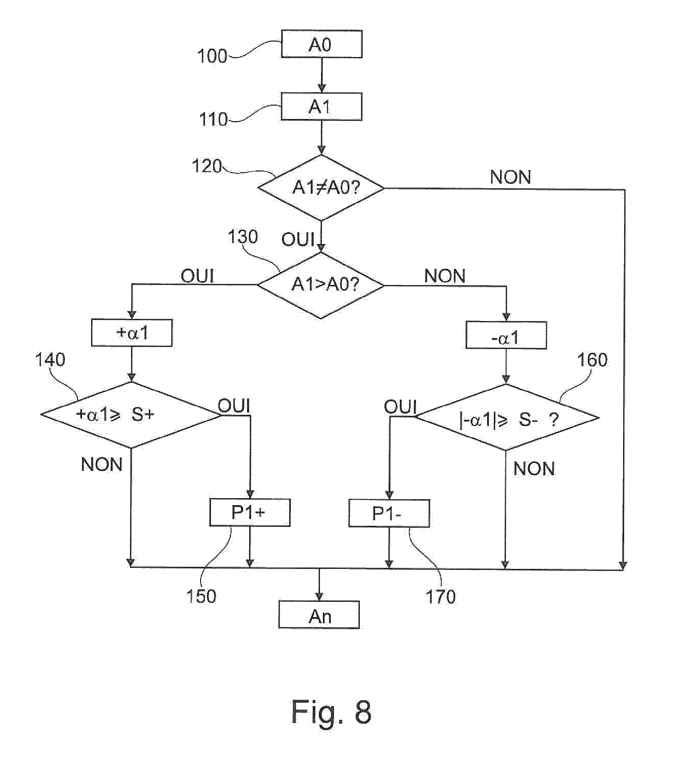

La figure 8 illustre maintenant la phase de démarrage de l'UC 10 selon l'invention, et en particulier la première boucle de modulation. Dans cet exemple, on considère que l'UC 10 démarre avec un paramètre de réglage P1 courant prédéterminé, par exemple la dimension de la tâche d'éclairement 3.

A l'étape 100, l'UC 10 relève une indication de position angulaire initiale A0 de la poignée 11 telle que par exemple celle illustrée par exemple à la figure 5. Cette position angulaire initiale A0 peut également être celle mémorisée à l'arrêt de l'UC 10 lors de l'utilisation précédente de l'appareil d'éclairage 1, ou encore correspondre à une position de référence dans laquelle la poignée 11 est repositionnée avant tout démarrage de l'UC 10.

A intervalles de temps n prédéterminés successifs, par exemple toutes les 5 ms après le démarrage à l'étape 100, l'UC 10 relève une indication de position angulaire courante de la poignée 11, ici la première position angulaire courante A1 telle que celle par exemple illustrée par la figure 6. Cette position angulaire courante A1 est enregistrée en mémoire dans l'UC 10.

A l'étape 120, l'UC 10 compare la position angulaire courante A1 à la position angulaire précédente c'est à dire la position angulaire initiale A0, laquelle est aussi maintenue en mémoire de l'UC 10.

Si à l'étape 120, la position angulaire courante A1 n'est pas différente de la position angulaire initiale A0, l'UC 10 maintient constant la valeur du paramètre de réglage P1 et le processus de traitement de données reboucle pour un nouvel intervalle de temps, ici de 5 ms. Dans le cas contraire, c'est-à-dire si à l'étape 120 la position angulaire courante A1 est différente de la position angulaire initiale A0, l'UC 10 calcule l'écart angulaire a1 entre les positions angulaires courante A1 et initiale A0. L'UC 10 détermine également, à l'étape 130, le sens de la rotation (+/-α1) de la poignée 11 entre les deux positions angulaires courante A1 et initiale A0, en particulier le sens de la rotation (+α1) si le sens de rotation de la poignée 11 est le sens horaire et le sens de la rotation (-α1) si le sens de rotation de la poignée 11 est le sens antihoraire.

Si à l'étape 130 l'UC 10 a déterminé un écart angulaire +α1 non nul dans le sens horaire, à l'étape 140, l'UC 10 compare cet écart angulaire +α1 à un premier seuil S+ prédéterminé (seuil horaire), ce seuil horaire S+ pouvant être enregistré en mémoire dans l'UC 10 pour être associé au paramètre de réglage P1. On doit comprendre qu'on peut avoir plusieurs seuils S+ différents associés respectivement à différents paramètres de réglage pouvant être modulés dans l'UC 10.

Si à l'étape 140, l'UC 10 détermine que l'écart angulaire +α1 a une valeur inférieure au seuil horaire S+, c'est à dire que la poignée 11 n'a pas assez tourné dans le sens horaire, alors l'UC 10 maintient constant la valeur du paramètre de réglage P1 et le processus de traitement de données reboucle pour un nouvel intervalle de temps. Si au contraire, à l'étape 140, l'UC 10 détermine que la valeur de l'écart angulaire +α1 est supérieure (ou égale) au seuil horaire S+, alors à l'étape 150, l'UC 10 module (fait varier) de façon prédéterminée la valeur du paramètre de réglage P1 et poursuit le traitement pour un nouvel intervalle de temps. La variation du paramètre réglage P1 est indiquée en 150 par P1+. Cette variation peut consister en une augmentation graduelle de la valeur, par exemple un centimètre de plus de diamètre pour la tâche d'éclairement 3.

Si à l'étape 130, l'UC 10 a déterminé un écart angulaire -α1 non nul dans le sens antihoraire, à l'étape 160, l'UC 10 compare cet écart angulaire - α1 à un second seuil S- prédéterminé (seuil antihoraire), ce seuil antihoraire S- pouvant être enregistré en mémoire dans l'UC 10 pour être associé aussi au paramètre de réglage P1.

On doit aussi comprendre qu'on peut avoir plusieurs seuils horaire S+ et seuils antihoraire S- différents associés respectivement à différents paramètres de réglage pouvant être modulés dans l'UC 10.

Si à l'étape 160, l'UC 10 détermine que l'écart angulaire -α1 a une valeur inférieure au seuil antihoraire S-, alors l'UC 10 maintient constant la valeur du paramètre de réglage P1 et le processus de traitement de données se poursuit pour un nouvelle intervalle de temps qui intervient à l'expiration de l'intervalle de temps courant n. Si au contraire, à l'étape 160, l'UC 10 détermine que la valeur de l'écart angulaire -α1 est supérieure (ou égale) au seuil antihoraire S-, alors à l'étape 170, l'UC 10 module (fait varier) de façon prédéterminée la valeur du paramètre de réglage P1 et poursuit le traitement pour un nouvel intervalle de temps. La variation du paramètre P1 est indiquée en 170 par P1-. Cette variation peut consister en une diminution graduelle de la valeur, par exemple un centimètre de moins de diamètre pour la tâche d'éclairement 3.

On comprend que les modulations aux étapes 150 et 170 du paramètre de réglage P1 vont normalement dans des sens opposés (augmentation/diminution).

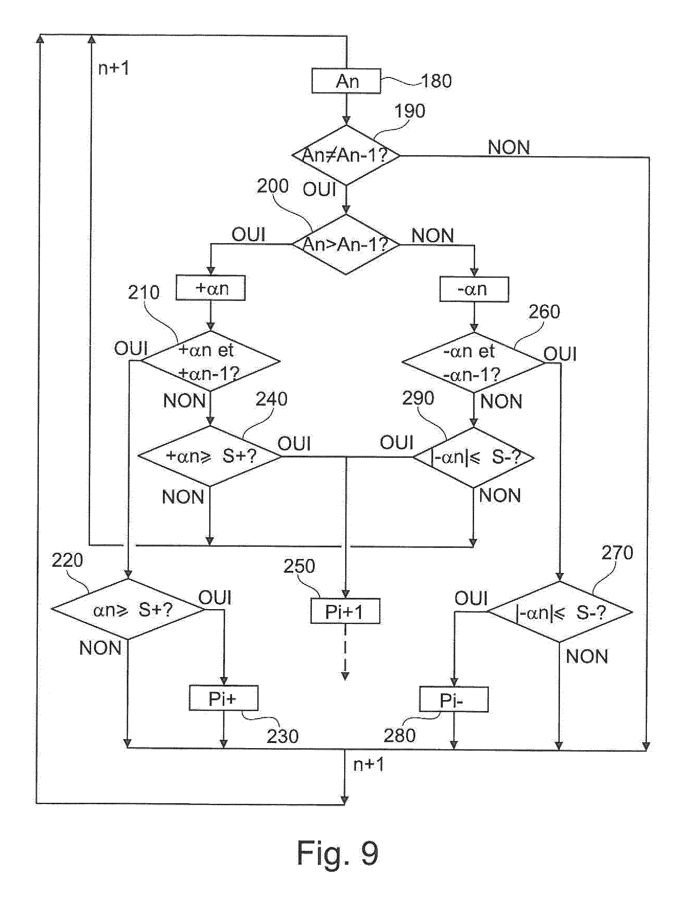

La figure 9 illustre une boucle de modulation subséquente à la première boucle de modulation illustrée sur la figure 8, c'est à dire à un traitement de données dans l'UC 10 qui correspond au n^{ième} intervalle de temps.

A l'étape 180, l'UC 10 relève la position angulaire courante An de la poignée 11 et l'enregistre en mémoire. A l'étape 190, l'UC 10 compare la position angulaire courante An avec la position angulaire précédente An-1 de la poignée 11 qui a été maintenue en mémoire dans l'UC 10, c'est à dire la position angulaire occupée par la poignée 11 à la fin de l'intervalle de temps précédent n-1, immédiatement avant l'intervalle de temps courant n. Si la position angulaire courante An est identique à la position angulaire précédente An-1 de la poignée 11, l'UC 10 maintient constant, comme indiqué plus haut, le paramètre de réglage courant activé dans l'UC 10 et indiqué par Pi et le processus se poursuit sur une nouvelle boucle de modulation pour un nouvel intervalle de temps.

Si au contraire à l'étape 90, la position angulaire courante An est différente de la position angulaire précédente An-1 de la poignée 11, l'UC 10 calcule à l'étape 200 l'écart angulaire αn entre les positions angulaires courante An et précédente An-1. L'UC 10 détermine également, à l'étape 200, le sens de la rotation de la poignée 11 entre les deux positions angulaires courante An et précédente An-1, en particulier le sens de la rotation (+αn) si le sens de rotation de la poignée 11 est le sens horaire et le sens de la rotation (-αn) si le sens de rotation de la poignée 11 est le sens antihoraire. Cet écart angulaire +/-αn courant avec le sens de rotation courant sont aussi enregistrés à l'étape 200 en mémoire par l'UC 10 à chaque intervalle de temps courant n.

Si à l'étape 200, l'UC 10 détermine que le sens de rotation pendant l'intervalle de temps courant n est le sens de rotation horaire (+αn), à l'étape 210, l'UC 10 contrôle si le sens de rotation précédent αn-1 lors de l'intervalle de temps précédent n-1 était également un sens de rotation horaire (+αn). Dans l'affirmative, à l'étape 220, l'UC 10 compare l'écart angulaire courant +αn au seuil horaire S+ prédéterminé associé au paramètre de réglage Pi courant. Si la valeur de l'écart angulaire courant +αn est inférieure au seuil horaire S+, alors l'UC 10 maintient constant la valeur du paramètre de réglage Pi (pas d'action de réglage sur l'appareil d'éclairage 1) et le processus reboucle pour un nouvel intervalle de temps n+1. Si au contraire, la valeur de l'écart angulaire courant +αn est supérieure (ou égale) au seuil horaire S+, alors l'UC 10 fait varier (incrémente) la valeur du paramètre de réglage Pi comme indiqué plus haut, et reboucle sur un nouvel intervalle de temps n+1.

Si maintenant, à l'étape 200, l'UC 10 détecte un changement de sens de rotation de la poignée 11 pendant les deux intervalles de temps courant n et précédent n-1, c'est à dire ici que le sens de rotation de la poignée 11 pour l'intervalle de temps précédent n-1 était le sens antihoraire (-αn-1), alors à l'étape 240, l'UC 10 compare l'écart angulaire courant +αn au seuil horaire S+ associé au paramètre de réglage Pi courant.

Si à l'étape 240 la valeur de l'écart angulaire courant +αn est inférieure au seuil horaire S+, l'UC 10 maintient constant la valeur du paramètre de réglage Pi courant (pas de variation du paramètre) et reboucle pour un nouvel intervalle de temps n+1 et si au contraire la valeur de l'écart angulaire courant +αn est supérieure (ou égale) au seuil horaire S+, l'UC 10 change le paramètre de réglage. Dans l'exemple, l'UC 10 passe au paramètre de réglage Pi+1 qui correspondant par exemple au réglage de l'intensité lumineuse de l'éclairage. Le processus reboucle ensuite sur un nouvel intervalle de temps n+1.

Si à l'étape 200, l'UC 10 détermine que le sens de rotation pendant l'intervalle de temps courant n est le sens de rotation antihoraire (-αn), à l'étape 260, l'UC 10 contrôle si le sens de rotation précédent αn-1 lors de l'intervalle de temps précédent n-1 était également un sens de rotation antihoraire (-αn-1). Dans l'affirmative, à l'étape 290, l'UC 10 compare l'écart angulaire courant -αn au seuil antihoraire S- prédéterminé associé au paramètre de réglage Pi courant. Si la valeur de l'écart angulaire courant -αn est inférieure au seuil antihoraire S-, alors l'UC 10 maintient constant la valeur du paramètre de réglage Pi (pas d'action de réglage sur l'appareil d'éclairage 1) et poursuit son traitement pour un nouvel intervalle de temps n+1. Si au contraire, la valeur de l'écart angulaire courant -αn est supérieure (ou égale) au seuil antihoraire S-, alors l'UC 10 fait varier (diminue) la valeur du paramètre de réglage Pi et reboucle sur un intervalle de temps n+1.

Si maintenant à l'étape 260, l'UC 10 détecte un changement de sens de rotation de la poignée 11 pendant les deux intervalles de temps courant n et précédent n-1, c'est à dire ici que le sens de rotation de la poignée 11 pour l'intervalle de temps précédent n-1 était le sens horaire (+αn-1), alors à l'étape 290, l'UC 10 compare l'écart angulaire courant -αn au seuil antihoraire S- prédéterminé associé au paramètre de réglage Pi courant.

Si à l'étape 290, la valeur de l'écart angulaire courant -αn est inférieure au seuil antihoraire S-, l'UC 10 maintient constant la valeur du paramètre de réglage Pi courant (pas de variation du paramètre) et reboucle pour un nouvel intervalle de temps n+1 et si au contraire la valeur de l'écart angulaire courant -αn est supérieure (ou égal)e au seuil antihoraire S-, l'UC 10 passe au paramètre de réglage Pi+1, par exemple en suivant un liste circulaire de paramètres de réglage comme indiqué plus haut, c'est à dire passe au paramètre de réglage Pi+1. Le processus reboucle ensuite sur un nouvel intervalle de temps n+1.

On comprend donc qu'une rotation dans un sens et dans l'autre sur deux intervalles de temps consécutifs peut être détectée par l'UC 10 et commander un changement du paramètre de réglage Pi contrôlé par la rotation de la poignée 11.

On comprend aussi que la variation d'un paramètre de réglage Pi se fait en tournant la poignée 11 dans le même sens sur deux intervalles de temps n-1, n, consécutifs et que cette variation est plutôt de type incrémentale ou graduelle. On peut aussi prévoir une gradation progressive plutôt que incrémentale sans sortir du cadre de l'invention.

A chaque paramètre de réglage Pi, on peut avantageusement affecter des seuils antihoraire S- et horaire S+ particuliers, le seuil antihoraire S-pouvant en plus être différent du seuil horaire S+. On peut donc avoir autant de couple de seuils antihoraire S- et horaire S+ qu'il y a de paramètres de réglage Pi sous contrôle dans l'UC 10.

A titre d'exemple, les seuils antihoraire S- et horaire S+ peuvent être chacun de 45° mais il est entendu que si on diminue ce niveau de seuil, on augmente d'autant la sensibilité de l'UC 10 au contrôle du paramètre de réglage Pi.

Selon l'invention, la modulation du paramètre de réglage Pi ne dépend pas d'une position angulaire absolue de la poigné 11 dans la coupole 7 mais d'un écart relatif de positions angulaires de la poignée 11 entre une position courante et une position précédente qu'elle occupait lors du relevé précédent. Ainsi, du fait d'un seuil S à atteindre entre ces deux positions angulaires, avant de déclencher toute modulation du paramètre de réglage Pi, l'appareil d'éclairage 1 selon l'invention permet de limiter la sensibilité de la commande de modulation. La poignée 11 peut ainsi continuer à être utilisée pour déplacer la coupole 7 sans provoquer de réglage involontaire.

De plus, l'absence de butée physique sur la course angulaire de la poignée 11 rend le réglage de l'appareil d'éclairage 1 opérationnel à tout moment, dès le franchissement du seuil S prédéterminé de rotation de la poignée 11. Enfin, étant donné les boucles de modulation répétées, la modulation du paramètre de réglage Pi reste efficace et précise.

La construction particulière du dispositif d'éclairage 1 selon l'invention permet en plus de décentrer le capteur de position angulaire 31 de l'axe de la poignée 11. La poignée 11 peut ainsi être creuse et recevoir dedans un dispositif de vision ou tout dispositif adapté.

Il va de soi que la présente invention ne saurait être limitée à la description qui précède d'un de ses modes de réalisation, susceptibles de subir quelques modifications sans pour autant sortir du cadre de l'invention.

## Revendications

1. Appareil d'éclairage (1), notamment pour champ opératoire (4), comprenant un module de lumière (8) avec une poignée (11) mobile en rotation et une unité de contrôle/commande (10) qui, en réponse à une rotation de ladite poignée (11), agit sur un réglage dudit appareil d'éclairage (1), ladite unité de contrôle/commande (10) étant agencée pour relever (180), à intervalles de temps (n-1, n) réguliers successifs, une indication de position angulaire relative (An-1, An) de ladite poignée (11) et agir sur ledit réglage à partir des indications de position angulaire relatives courante (An) et précédente (An-1) relevées respectivement pour un intervalle de temps courant (n) et pour un intervalle de temps (n-1) précédant immédiatement ledit intervalle de temps courant (n), **caractérisé en ce que** ladite unité de contrôle/commande (10) est agencée pour calculer (190) un écart angulaire courant (αn) à partir desdites indications de position angulaire courante (An) et précédente (An-1), et comparer (220, 270) ledit écart angulaire courant (αn) à un seuil (S+/-) prédéterminé et **en ce que** ladite unité de contrôle/commande (10) est agencée pour modifier la valeur d'un paramètre de réglage (Pi, Pi+1) dudit appareil d'éclairage (1) quand ledit écart angulaire courant (αn) est supérieur audit seuil (S+/-).

2. Appareil d'éclairage (1) selon la revendication 1, **caractérisé en ce que** ladite unité de contrôle/commande (10) est agencée pour déterminer (200), à chaque intervalle de temps courant (n), un sens pour ledit écart angulaire courant (+αn/+αn) et augmenter ou diminuer la valeur du paramètre de réglage (Pi, Pi+1) en fonction du sens de l'écart angulaire courant (+αn/+αn).

3. Appareil d'éclairage (1) selon la revendication 2, **caractérisé en ce que** ladite unité de contrôle/commande (10) est agencée pour comparer ledit 1 écart angulaire courant (+αn/+αn) à un premier seuil (S+) prédéterminé pour un premier sens de rotation (+αn) de ladite poignée (11) et pour comparer ledit écart angulaire courant (+αn/+αn) à un second seuil seuil (S-) prédéterminé pour un second sens de rotation (-αn) de la ladite poignée (11), opposé audit premier sens de rotation (+αn), ledit premier seuil (S+) étant différent dudit second seuil (S-).

4. Appareil d'éclairage (1) selon l'une des revendications précédentes, **caractérisé en ce que** ladite unité de contrôle/commande (10) est agencée pour détecter (210, 260) un changement du sens de rotation de ladite poignée (11) pendant ledit intervalle de temps courant (n) et ledit intervalle de temps précédent (n-1) et, en réponse à ladite détection, basculer (250) le réglage dudit appareil d'éclairage (1) d'un paramètre de réglage (Pi) courant sur un paramètre de réglage (Pi+1) prédéterminé.

5. Appareil d'éclairage (1) selon la revendication 2, **caractérisé en ce que** ladite valeur dudit paramètre de réglage (Pi, Pi+1) est une puissance d'éclairage, une température de couleur ou une dimension de tache lumineuse.

6. Appareil d'éclairage (1) selon la revendication 4, **caractérisé en ce que** ladite unité de contrôle/commande (10) bascule d'un paramètre de réglage (Pi) courant à un autre paramètre de réglage (Pi+1) suivant une liste circulaire prédéterminée de paramètres de réglage.

7. Appareil d'éclairage (1) selon l'une des revendications précédentes, **caractérisé en ce qu'**il comporte une roue dentée (20) coaxiale à ladite poignée (11) et liée en rotation à ladite poignée (11), un pignon (26) agencé pour engrener avec ladite roue dentée (20), et un capteur de position angulaire (31) relatif apte à détecter la position angulaire relative de ladite poignée (11) par l'intermédiaire de la position angulaire relative dudit pignon (26).

8. Appareil d'éclairage (1) selon la revendication 7, **caractérisé en ce que** ledit capteur de position angulaire (31) est de type potentiomètre.

9. Appareil d'éclairage (1) selon la revendication 7 **caractérisé en ce que** ladite poignée (11) est creuse et apte à loger un support connecteur vidéo (23).

## Patentansprüche

1. Beleuchtungseinrichtung (1), insbesondere für ein Operationsfeld (4), umfassend ein Lichtmodul (8) mit einem drehbeweglichen Griff (11) und eine Kontroll-/Steuereinheit (10), die als Antwort auf eine Drehung des Griffes (11) auf eine Regelung der Beleuchtungseinrichtung (1) einwirkt, wobei die Kontroll-/Steuereinheit (10) eingerichtet ist, um eine Anzeige einer relativen Winkelposition (An-1, An) des Griffes (11) in regelmäßigen aufeinanderfolgenden Zeitintervallen (n-1, n) fest zu stellen (180) und um auf die Regelung auf Basis der festgestellten Anzeigen von laufenden (An) und vorhergehenden (An-1) relativen Winkelpositionen für ein laufendes Zeitintervall (n) und für ein unmittelbar dem laufenden Zeitintervall (n) vorhergehendes Zeitintervall (n-1) einzuwirken, **dadurch gekennzeichnet, dass** die Kontroll-/Steuereinheit (10) eingerichtet ist, um eine laufende Winkelabweichung (αn) aus den Anzeigen laufender (An) und vorhergehender (An-1) Winkelpositionen zu berechnen (190) und um die laufende Winkelabweichung (αn) mit einer vorbestimmten Schwelle (S+/-) zu vergleichen (220, 270), und dass die Kontroll-/Steuereinheit (10) eingerichtet ist, um den Wert eines Regelparameters (Pi, Pi+1) der Beleuchtungseinrichtung (1) zu verändern, wenn die laufende Winkelabweichung (αn) größer als die Schwelle (S+/-) ist.

2. Beleuchtungseinrichtung (1) nach Anspruch 1, **dadurch gekennzeichnet, dass** die Kontroll-/Steuereinheit (10) eingerichtet ist, um in jedem laufenden Zeitintervall (n) eine Richtung für die laufende Winkelabweichung (+αn/-αn) zu bestimmen (200) und den Wert des Regelparameters (Pi, Pi+1) in Abhängigkeit von der Richtung der laufenden Winkelabweichung (+αn/-αn) zu vergrößern oder zu verkleinern.

3. Beleuchtungseinrichtung (1) nach Anspruch 2, **dadurch gekennzeichnet, dass** die Kontroll-/Steuereinheit (10) eingerichtet ist, um die laufende Winkelabweichung (+αn/-αn) mit einer ersten vorbestimmten Schwelle (S+) für eine erste Drehrichtung (+αn) des Griffes (11) zu vergleichen und die laufende Winkelabweichung (+αn/-αn) mit einer zweiten vorbestimmten Schwelle (S-) für eine zweite Drehrichtung (-αn) des Griffes (11), die zu der ersten Drehrichtung (+αn) entgegengesetzt ist, zu vergleichen, wobei die erste Schwelle (S+) zu der zweiten Schwelle (S-) unterschiedlich ist.

4. Beleuchtungseinrichtung (1) nach einem der vorhergehenden Ansprüche, **dadurch gekennzeichnet, dass** die Kontroll-/Steuereinheit (10) eingerichtet ist, um eine Änderung der Drehrichtung des Griffes (11) während des laufenden Zeitintervalls (n) und des vorhergehenden Zeitintervalls (n-1) zu erfassen (210, 260) und als Antwort auf die Erfassung die Regelung der Beleuchtungseinrichtung (1) von einem laufenden Regelparameter (Pi) auf einen vorbestimmten Regelparameter (Pi+1) um zu stellen (250).

5. Beleuchtungseinrichtung (1) nach Anspruch 2, **dadurch gekennzeichnet, dass** der Wert des Regelparameters (Pi, Pi+1) eine Beleuchtungsleistung, eine Farbtemperatur oder eine Lichtfleckdimension ist.

6. Beleuchtungseinrichtung (1) nach Anspruch 4, **dadurch gekennzeichnet, dass** die Kontroll-/Steuereinheit (10) von einem laufenden Regelparameter (Pi) auf einen anderen Regelparameter (Pi+1) gemäß einer vorbestimmten Umlaufliste von Regelparametern umstellt.

7. Beleuchtungseinrichtung (1) nach einem der vorhergehenden Ansprüche, **dadurch gekennzeichnet, dass** sie ein Zahnrad (20) koaxial zum Griff (11) und in Drehverbindung mit dem Griff (11), ein Ritzel (26), das dazu vorgesehen ist, in das Zahnrad (20) einzugreifen, und einen Fühler (31) für die relative Winkelposition umfasst, der geeignet ist, die relative Winkelposition des Griffes (11) mit Hilfe der relativen Winkelposition des Ritzeis (26) zu erfassen.

8. Beleuchtungseinrichtung (1) nach Anspruch 7, **dadurch gekennzeichnet, dass** der Winkelpositionsfühler (31) vom Typ Potentiometer ist.

9. Beleuchtungseinrichtung (1) nach Anspruch 7, **dadurch gekennzeichnet, dass** der Griff (11) hohl und geeignet ist, einen Videoanschlussträger (23) aufzunehmen.

## Claims

1. A lighting appliance (1), in particular for illuminating an operating field (4), the lighting appliance comprising a light module (8) having a handle (11) mounted to turn, and a monitoring and control unit (10) that responds to said handle (11) turning by acting on an adjustment setting of said lighting appliance (1), said monitoring and control unit (10) being arranged to read (180), at successive regular time intervals (n-1, n), an indication of the relative angular position (An-1, An) of said handle (12), and to act on said adjustment setting on the basis of the indications of the current relative angular position (An) and of the preceding relative angular position (An-1) that are read respectively for a current time interval (n) and for a time interval (n-1) immediately preceding said current time interval (n), said lighting appliance being **characterized in that** said monitoring and control unit (10) is arranged to compute (190) a current angular difference (αn) on the basis of said indications of the current angular position (An) and of the preceding angular position (An-1), and to compare (220, 270) said current angular difference (αn) with a predetermined threshold (S±), and **in that** said monitoring and control unit (10) is arranged to modify the value of an adjustment parameter (Pi, Pi+1) of said lighting appliance (1) when said current angular difference (αn) is greater than said threshold (S±).

2. A lighting appliance (1) according to claim 1, **characterized in that** said monitoring and control unit (10) is arranged to determine (200), at each current time interval (n), a direction for said current angular difference (+αn/-αn) and to increase or to decrease the value of the adjustment parameter (Pi, Pi+1) as a function of the direction of the current angular difference (+αn/-αn).

3. A lighting appliance (1) according to claim 2, **characterized in that** said monitoring and control unit (10) is arranged to compare said current angular difference (+αn/-αn) with a predetermined first threshold (S+) for a first turning direction (+αn) of said handle (11), and to compare said current angular difference (+αn/-αn) with a predetermined second threshold (S-) for a second turning direction (-αn) of said handle (11) that is opposite from said first turning direction (+αn), said first threshold (S+) being different from said second threshold (S-).

4. A lighting appliance (1) according to any preceding claim, **characterized in that** said monitoring and control unit (10) is arranged to detect (210, 260) a change of turning direction of said handle (11) during said current time interval (n) and said preceding time interval (n-1), and to respond to said detection by changing over (250) the adjustment of said lighting appliance (1) from a current adjustment parameter (Pi) to a predetermined adjustment parameter (Pi+1).

5. A lighting appliance (1) according to claim 2, **characterized in that** said value of said adjustment parameter (Pi, Pi+1) is an illumination power, a color temperature, or a light spot size.

6. A lighting appliance (1) according to claim 4, **characterized in that** said monitoring and control unit (10) changes over from a current adjustment parameter (Pi) to another adjustment parameter (Pi+1) following a predetermined circular list of adjustment parameters.

7. A lighting appliance (1) according to any preceding claim, **characterized in that** it has a toothed wheel (20) that is on the same axis as said handle (11) and that is constrained to turn with said handle (11), a pinion (26) arranged to mesh with said toothed wheel (20), and a relative angular position sensor (31) suitable for detecting the relative angular position of said handle (11) via the relative angular position of said pinion (26).

8. A lighting appliance (1) according to claim 7, **characterized in that** said angular position sensor (31) is of the potentiometer type.

9. A lighting appliance (1) according to claim 7, **characterized in that** said handle (11) is hollow and is suitable for receiving a video connector support (23).
